(19) Europäisches Patentamt
European Patent Office
Office européen des brevets

(11) Veröffentlichungsnummer: 0 144 892
B1

# (12) EUROPÄISCHE PATENTSCHRIFT

(45) Veröffentlichungstag der Patentschrift: **02.11.88**

(51) Int. Cl.⁴: **C 07 D 207/22**, C 07 D 211/72, A 61 K 31/395

(21) Anmeldenummer: **84114300.1**

(22) Anmeldetag: **27.11.84**

(54) **Bisamidindiphenylderivate, ein Verfahren zu ihrer Herstellung und ihre Verwendung als chemotherapeutika.**

(30) Priorität: **03.12.83 DE 3343815**

(43) Veröffentlichungstag der Anmeldung: **19.06.85 Patentblatt 85/25**

(45) Bekanntmachung des Hinweises auf die Patenterteilung: **02.11.88 Patentblatt 88/44**

(84) Benannte Vertragsstaaten: **AT BE CH DE FR GB IT LI LU NL SE**

(56) Entgegenhaltungen:
**EP-A-0 111 095**
**CH-A-553 776**
**DE-A-3 220 828**
**US-A-3 658 958**

(73) Patentinhaber: **HOECHST AKTIENGESELLSCHAFT, Postfach 80 03 20, D-6230 Frankfurt am Main 80 (DE)**

(72) Erfinder: **Venugopalan, Bindamadhavan, Dr., D-206, Usha Nagar, Bhandup Bombay 400 078 (IN)**
Erfinder: **Patel, Bomi, Dr., 'Maya' Tirandas Road, Powai Bombay 400 076 (IN)**
Erfinder: **Chatterjee, Dipak Kumar, Dr., G-21, Hoechst Officer's Quaters, Mulund Bombay 400 082 (IN)**
Erfinder: **Ganguli, Bimal Naresh, Dr., "Saurayuth" 173 Central Avenue, Chembur Bombay 400 071 (IN)**
Erfinder: **de Souza, Noel John, Dr., 702 Avanti Central Avenue, Santa Cruz(West) Bombay 400 054 (IN)**

**EP 0 144 892 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue chemotherapeutisch wirksame Bisamidindiphenylderivate der allgemeinen Formel I

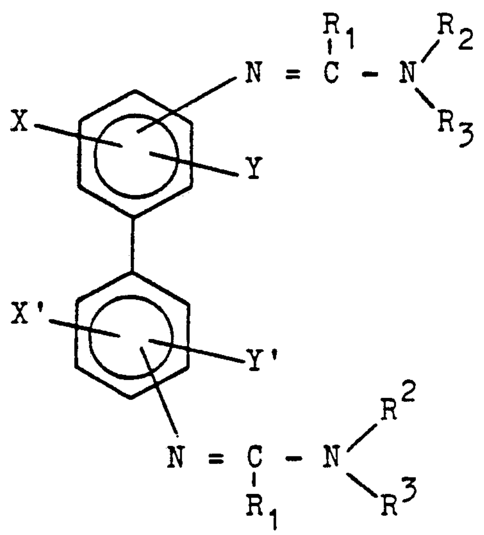

(I)

worin

| | |
|---|---|
| X und X' | jeweils $C_1$-$C_5$-Alkyl, $C_1$-$C_5$-Alkoxy, Halogen oder eine $SO_2CH_3$-Gruppe, |
| Y und Y' | jeweils Wasserstoff oder Halogen, |
| $R_1$ | Wasserstoff, $C_1$-$C_5$-Alkyl oder substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, |
| $R_2$ und $R_3$ | gleich oder verschieden sind und jeweils Wasserstoff, $C_1$-$C_5$-Alkyl, $C_1$-$C_5$-Alkanoyl oder substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, |
| $R_2$ und $R_3$ | zusammen mit dem N-Atom, an das sie gebunden sind, den Pyrrolidino-, Piperidino-, Imidazolino- oder Pyrimidino-Rest, oder |
| $R_1$ und $R_2$ | zusammen mit dem Kohlenstoffatom und dem Stickstoffatom, an das sie gebunden sind, den Pyrrolidino-, Piperidino-, Imidazolino- oder Pyrimidino-Rest, welcher ein- oder mehrfach substituiert sein kann mit $C_1$-$C_5$-Alkanoyl, $C_1$-C -Alkyl oder unsubstituiertem oder mit Halogen $NO_2$, CN, $CF_3$ oder $SO_3H$ substituiertem Phenyl, |
| $R_3$ | Wasserstoff, $C_1$-$C_5$-Alkyl, substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, $C_1$-$C_5$-Alkanoyl, $C_2$-$C_5$-Carbalkoxy oder unsubstituiertes oder mit Halogen, $NO_2$, CN, $CF_3$ oder $SO_3H$ substituieres Phenyl, |

bedeuten, deren Stereoisomere und pharmazeutisch verträgliche Salze und ein Verfahren zu ihrer Herstellung.

In der deutschen Offenlegungsschrift P 32 20 828 sind ähnliche Verbindungen der allgemeinen Formel I beschrieben, worin die Phenylringe jedoch mit elektrophilen Resten wie $NO_2$, CN, $CF_3$ und $SO_3H$ substituiert sind.

In der allgemeinen Formel I der Erfindung sind die folgenden Substituenten bevorzugt:

Falls $R_1$, $R_2$ und $R_3$ für Alkylgruppen stehen, ist $C_1$-$C_3$-Alkyl, also Methyl, Ethyl, n-Propyl oder Isopropyl bevorzugt.

Geeignete Salze der erfindungsgemäßen Bisamidinderivate sind beispielsweise solche von anorganischen oder organischen Säuren wie Hydrochloride, Hydrojodide, Hydrofluoride, Sulfonate, Acetate, Oxalate, Tartrate, Citrate, Maleate, Fumarate oder Methansulfonate.

Besonders bevorzugt sind substituierte 4,4'-Bisamidindiphenylderivate der allgemeinen Formel I'

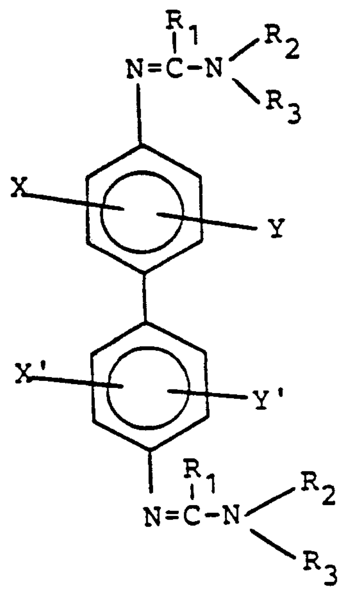

(I').

worin

| | |
|---|---|
| X und X' | jeweils $C_1$-$C_3$-Alkyl, $C_1$-$C_3$-Alkoxy oder Halogen, |
| >t Y und Y' | jeweils Wasserstoff oder Halogen, |
| $R_1$ | Wasserstoff, $C_1$-$C_3$-Alkyl oder substituiertes Alkyl der Formel $(CH_2)_nXR_4$, worin n eine Zahl von 1 - 3 bedeutet, X für O oder S steht und $R_4$ Wasserstoff, $C_1$-$C_3$-Alkyl oder $C_1$-$C_5$-Alkanoyl bedeutet, |
| $R_2$ und $R_3$ | jeweils $C_1$-$C_3$-Alkyl, oder |
| $R_2$ und $R_3$ | zusammen mit dem Stickstoffatom, an das sie gebunden sind, den Pyrrolidino-, Piperidino-, Imidazolino- oder Pyrimidino-Rest, |
| $R_1$ und $R_2$ | zusammen mit dem Kohlenstoffatom und dem Stickstoffatom, an das sie gebunden sind den Pyrrolidino-, Piperidino-, Imidazolino- oder Pyrimidino-Rest bedeuten, welcher mit $C_1$-$C_3$-Alkyl, $C_1$-$C_3$-Alkanoyl oder unsubstituiertem oder mit Halogen, $NO_2$, CN, $CF_3$ oder $SO_3H$ substituiertem Phenol substituiert sein können. |

sowie deren pharmazeutisch verträgliche Salze. Als erfindungsgemäße Verbindungen kommen insbesondere die folgenden in Betracht:

3,3'-Dichlor-4,4'-di-(pyrrolidin-2-ylidenamino)-diphenyl.
3,3'-Dichlor-4,4'-di-(pyrrolidin-2-ylidenamino)-diphenyl-dihydrochlorid-trihydrat.
3,3'-Dichlor-4,4'-di-(piperidin-2-ylidenamino)-diphenyl-hemihydrat.
3,3'-Dimethoxy-4,4'-di-(pyrrolidin-2-ylidenamino)-diphenyl-hemihydrat.
3,3'-Dimethoxy-4,4'-di-(piperidin-2-ylidenamino)-diphenyl-hemihydrat.
3,3'-Dimethyl-4,4'-di-(piperidin-2-ylidenamino)-diphenyl-hemihydrat.
4,4'-Di(piperidin-2-ylidenamino)-2,2', 5,5'-tetrachlor-diphenyl-hemihydrat.
3,3'-Dibrom-4,4'-di(pyrrolidin-2-ylidenamino)-diphenyl.
3,3-Dibrom-4,4'-di(piperidin-2-ylidenamino)-diphenyl.
3,3'-Difluoro-4,4'-di(pyrrolidin-2-ylideneamino)-diphenyl.
3,3'-Difluoro-4,4'-di(5-methylpyrrolidin-2-ylidenamino)-diphenyl.
In der folgenden Tabelle sind einige neue 4,4-Bisamidin-diphenylderivate aufgeführt:

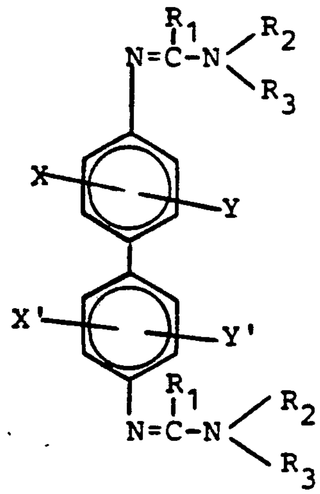

(I')

| X/X' | Y/Y' | $R_1$ $R_2$ | $R_3$ | Salz |
|---|---|---|---|---|
| Schmelzpunkt °C | | | | |
| 3-F/3'-F | H | $(CH_2)_3$ | H | - |
| 251 - 253 | 3-Br/3'-Br | H | $(CH_2)_3$ | H |
| - | 245 - 47 | 3-Cl/3'-Cl | H | $(CH_2)_3$ |
| H | - | 239 - 41 | 3-Cl/3' | HCl |
| $(CH_2)_3$ | H | $2HCl \cdot 3H_2O$ | > 300 | 3-Br/3'-Br |
| H | $(CH_2)_4$ | H | - | 244 - 46 |
| 3-Cl/3'-Cl | H | $(CH_2)_4$ | H | $1/2H_2O$ |
| 238 - 41 | $3-CH_3/3'-CH_3$ | H | $(CH_2)_3$ | H |
| $1/2H_2O$ | 231 - 34 d | $3-OCH_3/3'-OCH_3$ | H | $(CH_2)_3$ |
| H | $1/2H_2O$ | 223 - 26 | $3-CH_3/3'-CH_3$ | |
| $(CH_2)_4$ | H | $1/2H_2O$ | 225 - 28 | $3-OCH_3/3'-OCH_3$ |
| H | $(CH_2)_4$ | H | $1/2H_2O$ | 208 - 10 |
| 2-Cl/2'-Cl | 5-Cl/5'Cl | $(CH_2)_3$ | H | $1/2H_2O$ |
| 275 - 77 | 2-Cl/2'-Cl | 5-Cl/5'Cl | $(CH_2)_4$ | H |
| $1/2H_2O$ | 272 - 75 | 2-F/2'-F | H | $-(CH_2)_3-$ |
| H | - | 215 - 18 | 4-Cl/4' | HCl |
| $-(CH_2)_3-$ | H | - | 215 - 18 | 3-F/3'-F |
| H | $-(CH_2)_4-$ | H | - | 268 - 71 d |
| 3-Br/3'-Br | H | $-(CH_2)_5-$ | H | - |
| 252 - 54 d | 3-F/3'-F | H | $-(CH_2)CH(CH_3)-$ | |
| - | 251 - 53 d | 3-Cl/3'-Cl | H | $-(CH_2)CH(CH_3)-$ |
| H | - | 220 - 22 d | 3-F/3'-F | H |
| $-(CH_2)_2C(CH_3)_2$ | H | - | | 300 3-F/3'-F |
| H | $-CH_2CH(Ph)CH_2-$ | H | - | 230 - 32 d |
| $3-CH_3/3'-CH_3$ | $5-CH_3$ | $-(CH_2)_3-$ | H | - |
| 258 - 60 d | | $5'-CH_3$ | | |
| | | $3-CH_3/3'-CH_3$ | $5-CH_3$ | $-(CH_2)_4-$ |
| H | - | 242 - 44 d | | $5-CH_3$ |
| | | | | 3-F/3'-F |
| H | $-(CH_2)_3-$ | $CH_3$ | - | 168 - 70 d |
| 3-F/3'-F | H | $-(CH_2)CH(C_2H_5)$ | H | - |
| 238 - 40 d | 3-F/3'-F | H | $-OH(CH_3)CH_2C(OH_3)_2$ | |
| - | 222 - 24 d | | | |

(d = schmilzt unter Zersetzung)

Die Erfindung betrifft ferner ein Verfahren zur Herstellung von Bisamidindiphenylderivaten der allgemeinen Formel I sowie deren pharmazeutisch verträglichen Salze, das dadurch gekennzeichnet ist, daß entsprechend substituierte Benzidine mit Phosphoroxychlorid sowie entsprechend substituierten Amiden der allgemeinen Formel III $R_1CNR_2R_3$ zu

einer Verbindung der allgemeinen Formel I umgesetzt werden, die gegebenenfalls mittels einer Säure, wie Salzsäure, Jodwasserstoffsäure, Fluorwasserstoffsäure, Schwefelsäure, Phosphorsäure, Essigsäure, Oxalsäure, Weinsäure, Citronensäure, Maleinsäure, Fumarsäure oder Methansulfonsäure, in ihre Säureadditionssalze übergeführt wird.

Diese Reaktion kann durch Erhitzen des Reaktionsgemisches, beispielsweise auf Temperaturen von 60 bis über 100°C beschleunigt oder vervollständigt werden. Gegebenenfalls kann auch in Lösungsmitteln, wie Acetonitril oder in Äthern, wie Dioxan oder Tetrahydrofuran, gearbeitet werden.

Das Reaktionsprodukt der Verbindung der allgemeinen Formel I erhält man durch Verdünnen des Reaktionsgemisches mit Eiswasser, Basischstellen der wäßrigen Lösung, Abfiltrieren des entsprechenden Niederschlages und chromatographische Reinigung und Umkristallisierung aus Lösungsmitteln, wie beispielsweise ein Chloroform-Methanol-Gemisch (1 : 4) oder aus Chloroform: Petroleumäther. Gegebenenfalls kann die Verbindung in ihr Säureadditionssalz übergeführt werden und aus Lösungsmitteln, wie Ethanol-Äther umkristallisiert werden.

Die erfindungsgemäßen substituierten Bisamidindiphenylderivate der allgemeinen Formel I besitzen wertvolle chemotherapeutische Eigenschaften. Beispielsweise sind sie gegen Amöben und Trichomonaden wirksam, wie am Goldhamster und der Wistar-Ratte gezeigt werden konnte. Die wirksame Dosis beträgt 10 bis 300 mg/kg, vorzugsweise 25 bis 200 mg/kg, verabreicht über 5 Tage (oral oder parenteral).

Die Erfindung wird anhand der nachfolgenden Beispiele näher erläutert.

**Beispiel 1**

3,3'-Dichlor-4,4'-di-(pyrrolidin-2-ylidenamino)-diphenyl

Zu 2-Pyrrolidon (69 g) tropft man unter Rühren 16,8 ml Phosphoroxychlorid, wobei man durch äußere Kühlung dafür sorgt, daß eine Temperatur von 35 - 40°C nicht überschritten wird. Nach beendeter Zugabe rührt man das Reaktionsgemisch 1 Std. lang bei Zimmertemperatur und gibt dann 3,3'Dichlorbenzidin (14,5 g) zu. Dann erhitzt man das Reaktionsgemisch auf 110°C und beläßt es 3 Std. bei dieser Temperatur. Das noch heiße Reaktionsgemisch wird in kaltes Wasser gegossen. Die dabei erhaltene Lösung wird filtriert, und der pH-Wert des klaren Filtrats wird mit 28 %-iger Ammoniaklösung auf 9 eingestellt. Das so ausgefällte Produkt wird abgetrennt, mit Wasser gewaschen, getrocknet und durch Umkristallisieren aus Chloroform, das ein Gemisch aus 2 %-igem Methanol und Petroleumäther (60 - 80°C) enthält, gereinigt, wobei die gewünschte Verbindung in einer Menge von 15 g anfällt.

Schmp. 239 - 241°C.

**Beispiel 2**

3,3'-Dichlor-4,4'-di-(piperidin-2-ylidenamino)-diphenyl

Wie Beispiel 1, jedoch unter Verwendung von 2-Piperidon anstelle von 2-Pyrrolidon. Ausbeute: 46 %, Schmp. 238 - 241°C [Chloroform-Methanol (2 %), Petroleumäther 60 - 80°C].

**Beispiel 3**

3,3'-Dimethoxy-4,4'-di-(pyrrolidin-2-ylidenamino)-diphenyl-semihydrat

Wie Beispiel 1, jedoch unter Verwendung von 3,3'-Dimethoxybenzidin anstelle von 3,3'-Dichlorbenzidin. Ausbeute: 16 %, Schm. 231 - 234°C Zers. (Chloroform: Petroleumäther 60 - 80°C).

**Beispiel 4**

3,3'-Dimethoxy-4,4'-di-(piperidin-2-ylidenamino)-diphenyl-semihydrat

Wie Beispiel 1, jedoch unter Verwendung von 2-Piperidon anstelle von 2-Pyrrolidon und von 3,3'-Dimethoxybenzidin anstelle von 3,3'-Dichlorbenzidin. Ausbeute an der Titelverbindung 25 %, Schm. 225 - 228°C (Chloroform: Petroleumäther, 60 - 80°C).

**Beispiel 5**

3,3'-Dimethyl-4,4'-di-(piperidin-2-ylidenamino)-diphenyl-semihydrat
Wie Beispiel 1, jedoch unter Verwendung von 2-Piperidon anstelle von 2-Pyrrolidon und von 3,3'-Dimethylbenzidin anstelle von 3,3'-Dichlorbenzidin. Ausbeute an der gewünschten Verbindung 13 %, Schm. 225 - 228°C. (Chloroform: Petroleumäther 60 - 80°C).

**Beispiel 6**

4,4'-Bis-(pyrrolidin-2-ylidenamino)-2,2', 5,5'-tetrachlor-diphenyl-semihydrat
Wie Beispiel 1, jedoch unter Verwendung von 2,2', 5,5'-tetrachlorbenzidin anstelle von 3,3'-Dichlorbenzidin wobei die Titelverbindung in einer Ausbeute von 18 % erhalten wird. Schm. 275 - 277°C (Chloroform: Petroleumäther 60 - 80°C).

**Beispiel 7**

3,3'-Dibrom-4,4'-di-(pyrrolidin-2-ylidenamino)-diphenyl
Wie Beispiel 1, jedoch unter Verwendung von 3,3'-Dibrom-benzidin anstelle von 3,3'-Dichlorbenzidin. Ausbeute: 36 %, Schm. 245 - 247°C.

**Beispiel 8**

3,3'-Dibrom-4,4'-di-(piperidin-2-ylidenamino)-diphenyl
Wie Beispiel 1, jedoch unter Verwendung von 2-Piperidon anstelle von 2-Pyrrolidon und von 3,3'-Dichlorbenzidin. Die gewünschte Verbindung erhält man in einer Ausbeute von 40 %. Schm. 244 - 246°C.

**Beispiel 9**

3,3'-Dichlor-4,4'-di-(pyrrolidin-2-ylidenamino)-diphenyl-dihydrochlorid-trihydrat
Man löst das wie unter Beispiel 1 beschrieben hergestellte 3,3'-Dichlor-4,4'-di(pyrrolidon-2-ylidenamino)-diphenyl (1,0 g) in Methylenchlorid (15 ml), das Methanol (1 %) enthält, auf und gibt ätherische Salzsäure zu. Das dabei ausgefällte Hydrochlorid filtriert man, man trocknet das Filtrat und kristallisiert es aus einer Mischung aus Methylenchlorid, Methanol (1 %) und Äther um. Ausbeute: 90 %, Schm. 300°C.

**Beispiel 10**

3,3'-Difluor-4,4'-di-(pyrrolidin-2-ylidenamino)-diphenyl
Wie Beispiel 1, jedoch unter Verwendung von 3,3'-Difluorbenzidin anstelle von 3,3'-Dichlorbenzidin Ausbeute: 39 %, Schm. 251 - 253°C (Dimethylformamid)

**Patentansprüche** für die Vertragsstaaten BE CH DE FR GB IT LI LU NL SE

1. Verbindungen der allgemeinen Formel I

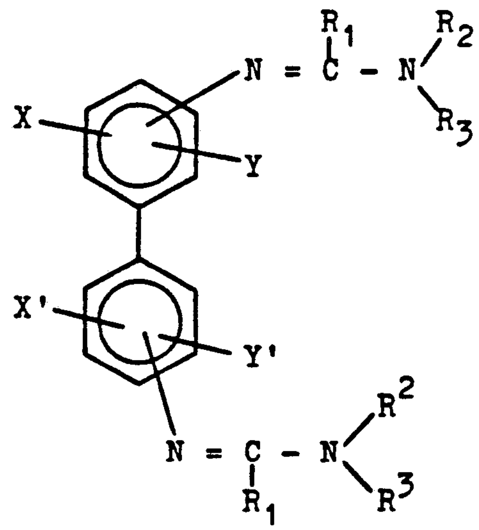

(I)

worin

| | |
|---|---|
| X und X' | jeweils $C_1$-$C_5$-Alkyl, $C_1$-$C_5$-Alkoxy, Halogen oder den Rest $SO_2CH_3$ |
| Y und Y' | jeweils Wasserstoff oder Halogen, |
| $R_1$ | Wasserstoff, $C_1$-$C_5$-Alkyl oder substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, |
| $R_2$ und $R_3$ | gleich oder verschieden sind und jeweils Wasserstoff, $C_1$-$C_5$-Alkyl, $C_1$-$C_5$-Alkanoyl oder substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, |
| $R_2$ und $R_3$ | zusammen mit dem N-Atom, an das sie gebunden sind, den Pyrrolidino-, Piperidino-, Imidazolino- oder Pyrimidino-Rest, oder |
| $R_1$ und $R_2$ | zusammen mit dem Kohlenstoffatom und dem Stickstoffatom, an das sie gebunden sind, den Pyrrolidino-, Piperidino-, Imidazolino- oder Pyrimidino-Rest, welcher ein- oder mehrfach substituiert sein kann mit $C_1$-$C_5$-Alkanoyl, $C_1$-$C_5$-Alkyl oder unsubstituiertem oder mit Halogen, $NO_2$, CN, $CF_3$ oder $SO_3H$ substituiertem Phenyl, |
| $R_3$ | Wasserstoff, $C_1$-$C_5$-Alkyl, substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, $C_1$-$C_4$-Alkanoyl, $C_2$-$C_5$-Carbalkoxy oder unsubstituiertes oder mit Halogen, $NO_2$, CN, $CF_3$ oder $SO_3H$ substituiertes Phenyl, |

bedeuten, deren Stereoisomere und pharmazeutisch verträgliche Salze.

2. Verbindungen gemäß Anspruch 1 mit der allgemeinen Formel I'

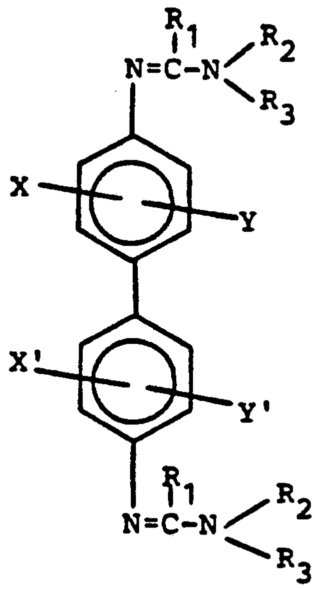

(I')

worin

X,X', Y,Y', $R_1$, $R_2$ und $R_3$ die in Anspruch 1 angegebene Bedeutung haben.

3. 3,3'-Dichlor-4,4'-di-(pyrrolidin-2-ylidenamino)-diphenyl.

4. 3,3'-Difluor-4,4'-di-(pyrrolidin-2-ylidenamino)-diphenyl.

5. 3,3-Difluor-4,4'-di-(5-methylpyrrolidin-2-ylidenamido)-diphenyl.

6. Verfahren zur Herstellung von Verbindungen der allgemeinen Formel I gemäß Anspruch 1, dadurch gekennzeichnet, daß man eine Verbindung der allgemeinen Formel II

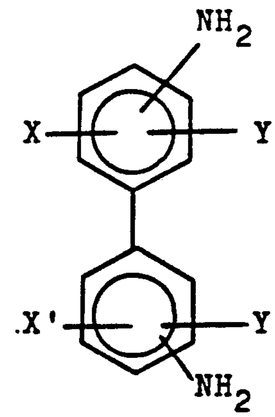

(II)

worin X, X', Y und Y' die in Anspruch 1 angegebene Bedeutung haben, mit Phosphoroxychlorid und einem Amid der allgemeinen Formel III

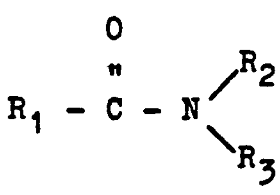

(III)

worin $R_1$, $R_2$ und $R_3$ die in Anspruch 1 angegebene Bedeutung haben, zu einer Verbindung der allgemeinen Formel I umsetzt und diese gegebenenfalls in ihr pharmazeutisch verträgliches Säureadditionssalz umwandelt.

7. Verfahren gemäß Anspruch 3, dadurch gekennzeichnet, daß die Reaktionsmischung auf 60 - 80°C erhitzt wird.

8. Verfahren gemäß Anspruch 4, dadurch gekennzeichnet, daß die Reaktion in einem halogenierten Kohlenwasserstoff oder einem Äther durchgeführt wird.

9. Arzneimittel, dadurch gekennzeichnet, daß es eine Verbindung der allgemeinen Formel I oder deren pharmakologisch verträgliches Säureadditionssalz in Kombination mit einem üblichen pharmazeutischen Träger und/oder Stabilisator enthält.

10. Verwendung einer Verbindung gemäß Anspruch 1 zur Herstellung eines Arzneimittels mit Wirksamkeit gegen Amöben und Trichomonaden.

**Patentansprüche** für den Vertragsstaat AT

1. Verfahren zur Herstellung von Verbindungen der allgemeinen Formel I

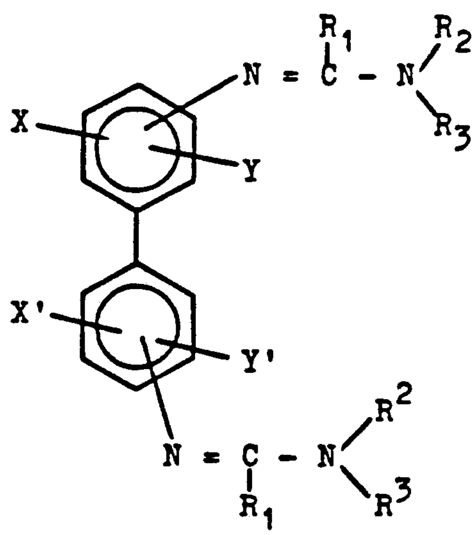

(I)

worin

| | |
|---|---|
| X und X' | jeweils $C_1$-$C_5$-Alkyl, $C_1$-$C_5$-Alkoxy, Halogen oder den Rest $SO_2CH_3$ |
| Y und Y' | jeweils Wasserstoff oder Halogen, |
| $R_1$ | Wasserstoff, $C_1$-$C_5$-Alkyl oder substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, |
| $R_2$ und $R_3$ | gleich oder verschieden sind und jeweils Wasserstoff, $C_1$-$C_5$-Alkyl, $C_1$-$C_5$-Alkanoyl oder substituiertes Alkyl der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, |
| $R_2$ und $R_3$ | zusammen mit dem N-Atom, an das sie gebunden sind, den Pyrrolidino-, Piperidino-, Imidazolino- oder Pyrimidino-Rest, oder |
| $R_1$ und $R_2$ | zusammen mit dem Kohlenstoffatom und dem Stickstoffatom, an das sie gebunden sind, den Pyrrolidino-, Piperidino-, Imidazolino- oder Pyrimidino-Rest, welche ein- oder mehrfach substituiert sein kann mit $C_1$-$C_5$-Alkanoyl, $C_1$-$C_5$-Alkyl oder unsubstituiertem oder mit Halogen, $NO_2$, CN, $CF_3$ oder $SO_3H$ substituiertem Phenyl |
| $R_3$ | Wasserstoff, $C_1$-$C_5$-Alkyl, substituiertes Alkyl, der Formel $(CH_2)_nXR_4$, worin n eine ganze Zahl von 1 - 3 bedeutet, X für O oder S steht und $R_4$ Wasserstoff, $C_1$-$C_3$-Alkyl oder $C_1$-$C_5$-Alkanoyl bedeutet, $C_1$-$C_5$-Alkanoyl, $C_2$-$C_5$-Carbalkoxy oder unsubstituiertes oder mit Halogen, $NO_2$, CN, $CF_3$ oder $SO_3H$ substituiertes Phenyl, |

bedeuten, deren Stereoisomeren und pharmazeutisch verträglichen Salzen,
dadurch gekennzeichnet, daß man
eine Verbindung der allgemeinen Formel II

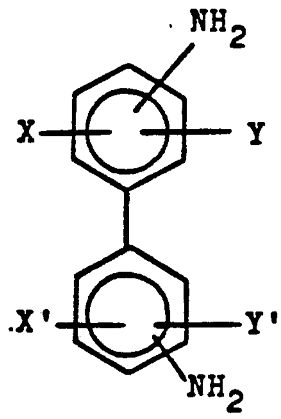

(II)

worin X, X', Y und Y' die oben angegebene Bedeutung haben, mit Phosphoroxychlorid und einem Amid der allgemeinen Formel III

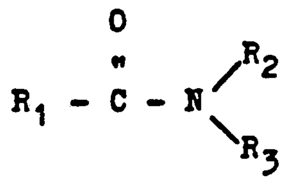

(III)

worin $R_1$, $R_2$ und $R_3$ die oben angegebene Bedeutung haben, zu einer Verbindung der allgemeinen Formel I umsetzt und diese gegebenenfalls in ihr pharmazeutisch verträgliches Säureadditionssalz umwandelt.

2. Verfahren gemäß Anspruch 1, dadurch gekennzeichnet, daß die Reaktionsmischung auf 60 - 80°C erhitzt wird.

3. Verfahren gemäß Anspruch 2, dadurch gekennzeichnet, daß die Reaktion in einem halogenierten Kohlenwasserstoff oder einem Äther durchgeführt wird.

4. Verfahren gemäß den Ansprüchen 1 - 3, dadurch gekennzeichnet, daß eine Verbindung der allgemeinen Formel I hergestellt wird

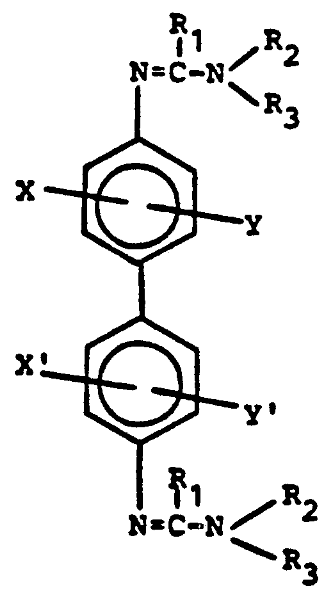

(I')

worin

X,X', Y,Y', $R_1$, $R_2$ und $R_3$ die in Anspruch 1 angegebene Bedeutung haben.

5. Verfahren gemäß Anspruch 4, dadurch gekennzeichnet, daß 3,3'-Dichlor-4,4'-di-(pyrrolidin-2-ylidenamino)-diphenyl hergestellt wird.

6. Verfahren gemäß Anspruch 4, dadurch gekennzeichnet, daß 3,3'-Difluor-4,4'-di-(pyrrolidin-2-ylidenamino)-diphenyl hergestellt wird.

7. Verfahren gemäß Anspruch 4, dadurch gekennzeichnet, daß 3,3'-Difluor-4,4'-di-(5-methyl-pyrrolidin-2-ylidenamino)diphenyl hergestellt wird.

8. Verfahren zur Herstellung von Arzneimitteln, dadurch gekennzeichnet, daß man eine Verbindung der allgemeinen Formel I oder deren pharmakologisch verträgliches Säureadditionssalz, mit pharmazeutisch üblichen Trägern und/oder Stabilisatoren, in eine therapeutisch geeignete Darreichungsform bringt.

9. Verwendung einer Verbindung gemäß Anspruch 1 zur Herstellung eines Arzneimittels mit Wirksamkeit gegen Amöben und Trichomonaden.

**Claims** for the contracting state: BE, CH, LI, DE, FR, GB, IT, LU, NL, SE

1. A compound of the general formula I

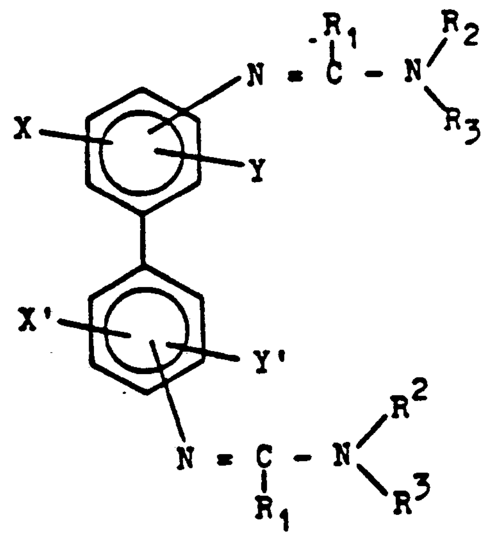

(I)

in which

| | |
|---|---|
| X and X' | each denote $C_1$-$C_5$-alkyl, $C_1$-$C_5$-alkoxy, halogen or the $SO_2CH_3$ radical, |
| Y and Y' | each denote hydrogen or halogen, |
| $R_1$ | denotes hydrogen, $C_1$-$C_5$-alkyl or substituted alkyl of the formula $(CH_2)_nXR_4$, in which n is an integer from 1 - 3, X represents O or S, and $R_4$ denotes hydrogen, $C_1$-$C_3$-alkyl or $C_1$-$C_5$-alkanoyl, |
| $R_2$ and $R_3$ | are identical or different and each denote hydrogen, $C_1$-$C_5$-alkyl, $C_1$-$C_5$-alkanoyl or substituted alkyl of the formula $(CH_2)_nXR_4$, in which n is an integer from 1 - 3, X represents O or S, and $R_4$ denotes hydrogen, $C_1$-$C_3$-alkyl or $C_1$-$C_5$-alkanoyl, |
| $R_2$ and $R_3$, | together with the N atom to which they are bonded, denote the pyrrolidino, piperidino, imidazolino or pyrimidino radical, or |
| $R_1$ and $R_2$, | together with the carbon atom and the nitrogen atom to which they are bonded, denote the pyrrolidino, piperidino, imidazolino or pyrimidino radical, which can be monosubstituted or polysubstituted by $C_1$-$C_5$-alkanoyl, $C_1$-$C_5$-alkyl or by phenyl which is unsubstituted or substituted by halogen, $NO_2$, CN, $CF_3$ or $SO_3H$, and |
| $R_3$ | denotes hydrogen, $C_1$-$C_5$-alkyl, substituted alkyl of the formula $(CH_2)_nXR_4$, in which n is an integer from 1 - 3, X represents O or S, and $R_4$ denotes hydrogen, $C_1$-$C_3$-alkyl or $C_1$-$C_5$-alkanoyl, or denotes $C_1$-$C_5$-alkanoyl, $C_2$-$C_5$-carbalkoxy or phenyl which is unsubstituted or substituted by halogen, $NO_2$, CN, $CF_3$ or $SO_3H$, |

and stereoisomers and pharmaceutically acceptable salts thereof.

2. A compound as claimed in claim 1, of the general formula I'

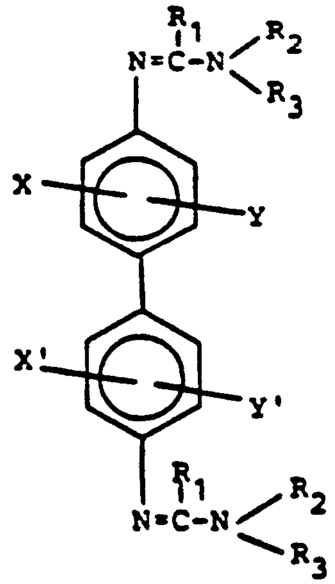

(I')

in which
X, X', Y, Y', $R_1$, $R_2$ and $R_3$ have the meaning given in claim 1.

3. 3,3'-Dichloro-4,4'-di-(pyrrolidin-2-ylideneamino)-diphenyl.
4. 3,3'-Difluoro-4,4'-di-(pyrrolidin-2-ylideneamino)-diphenyl.
5. 3,3-Difluoro-4,4'-di-(5-methylpyrrolidin-2-ylideneamido)-diphenyl.
6. A process for the preparation of a compound of the general formula I as claimed in claim 1, which comprises reacting a compound of the general formula II

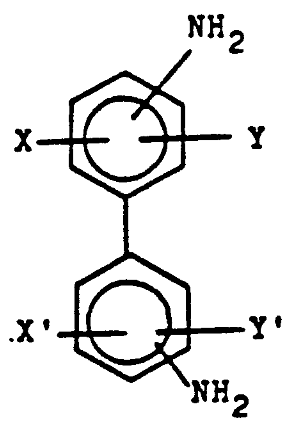

(II)

in which X, X', Y and Y' have the meaning given in claim 1, with phosphorus oxychloride and an amide of the general formula III

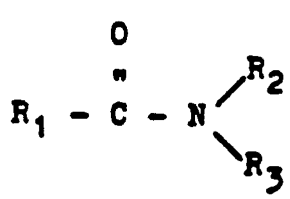

(III)

in which $R_1$, $R_2$ and $R_3$ have the meaning given in claim 1, to give a compound of the general formula I and, if appropriate, converting this into its pharmaceutically acceptable acid addition salt.

7. The process as claimed in claim 6, wherein the reaction mixture is heated at 60 - 80°C.
8. The process as claimed in claim 7, wherein the reaction is carried out in a halogenated hydrocarbon or an ether.
9. A medicament, which contains a compound of the general formula I or a pharmacologically acceptable

acid addition salt thereof, in combination with a customary pharmaceutical excipient and/or stabilizer.

10. The use of a compound as claimed in claim 1 for the preparation of a medicament with an activity against amoebas a.ıd trichomonads.

**Claims** for the contracting state AT

1. A process for the preparation of a compound of the general formula I

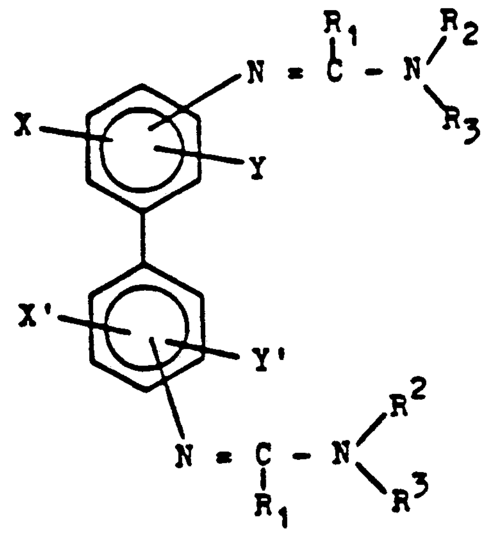

(I)

in which

| | |
|---|---|
| X and X' | each denote $C_1$-$C_5$-alkyl, $C_1$-$C_5$-alkoxy, halogen or the $SO_2CH_3$ radical, |
| Y and Y' | each denote hydrogen or halogen, |
| $R_1$ | denotes hydrogen, $C_1$-$C_5$-alkyl or substituted alkyl of the formula $(CH_2)_nXR_4$, in which n is an integer from 1 - 3, X represents O or S, and $R_4$ denotes hydrogen, $C_1$-$C_3$-alkyl or $C_1$-$C_5$-alkanoyl, |
| $R_2$ and $R_3$ | are identical or different and each denote hydrogen, $C_1$-$C_5$-alkyl, $C_1$-$C_5$-alkanoyl or substituted alkyl of the formula $(CH2)_nXR_4$, in which n is an integer from 1 - 3, X represents O or S, and $R_4$ denotes hydrogen $C_1$-$C_3$-alkyl or $C_1$-$C_5$-alkanoyl, |
| $R_2$ and $R_3$, | together with the N atom to which they are bonded, denote the pyrrolidino, piperidino, imidazolino or pyrimidino radical, or |
| $R_1$ and $R_2$, | together with the carbon atom and the nitrogen atom to which they are bonded, denote the pyrrolidino, piperidino, imidazolino or pyrimidino radical, which can be monosubstituted or polysubstituted by $C_1$-$C_5$-alkanoyl, $C_1$-$C_5$-alkyl or by phenyl which is unsubstituted or substituted by halogen, $NO_2$, CN, $CF_3$, or $SO_3H$, and |
| R3 | denotes hydrogen, $C_1$-$C_5$-alkyl, substituted alkyl of the formula $(CH_2)_nXR_4$, in which n is an integer from 1 - 3, X represents O or S, and $R_4$ denotes hydrogen, $C_1$-$C_3$-alkyl or $C_1$-$C_5$-alkanoyl, or denotes $C_1$-$C_5$-alkanoyl, $C_2$-$C_5$-carbalkoxy or phenyl which is unsubstituted or substituted by halogen, $NO_2$, CN, $CF_3$ or $SO_3H$, |

and stereoisomers and pharmaceutically acceptable salts thereof, which comprises reacting a compound of the general formula II

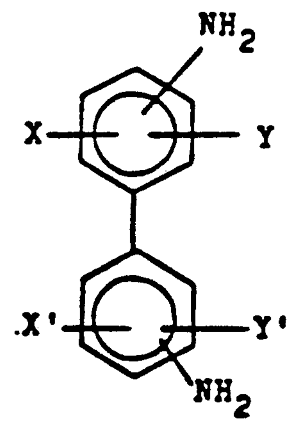

(II)

in which X, X', Y and Y' have the meaning given above, with phosphorus oxychloride and an amide of the general formula III

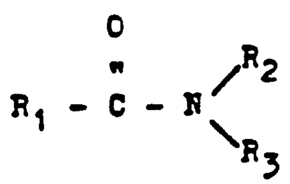

(III)

in which $R_1$, $R_2$ and $R_3$ have the meaning given above, to give a compound of the general formula I and, if appropriate, converting this into its pharmaceutically acceptable acid addition salt.

2. The process as claimed in claim 1, wherein the reaction mixture is heated at 60 - 80°C.

3. The process as claimed in claim 2, wherein the reaction is carried out in a halogenated hydrocarbon or an ether.

4. The process as claimed in any of claims 1 - 3, wherein a compound of the general formula I'

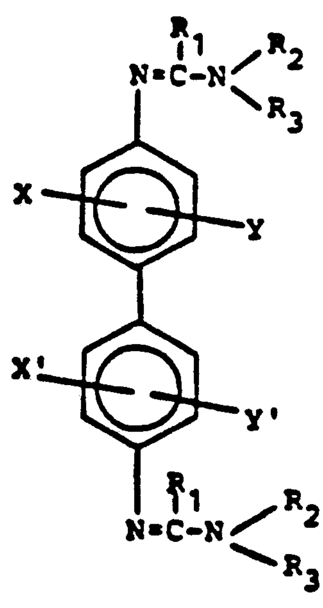

(I')

in which

X, X', Y, Y', $R_1$, $R_2$ and $R_3$ have the meaning given in claim 1, is prepared.

5. The process as claimed in claim 4, wherein 3,3'-dichloro-4,4'-di-(pyrrolidin-2-ylideneamino)-diphenyl is prepared.

6. The process as claimed in claim 4, wherein 3,3'-difluoro-4,4'-di-(pyrrolidin-2-ylidineamino)-diphenyl is

prepared.

7. The process as claimed in claim 4, wherein 3,3'-difluoro-4,4'-di-(5-methylpyrrolidin-2-ylidineamino)-diphenyl is prepared.

8. A process for the preparation of a medicament, which comprises bringing a compound of the general formula I or a pharmacologically acceptable acid addition salt thereof, into a therapeutically suitable administration form with a customary pharmaceutical excipient and/or stabilizer.

9. The use of a compound as claimed in claim 1 for the preparation of a medicament with an activity against amoebas and trichomonads.

**Revendications** pour les Etats contractants: BE, CH, LI, DE, FR, GB, IT, LU, NL, SE

1. Composés de formule générale I

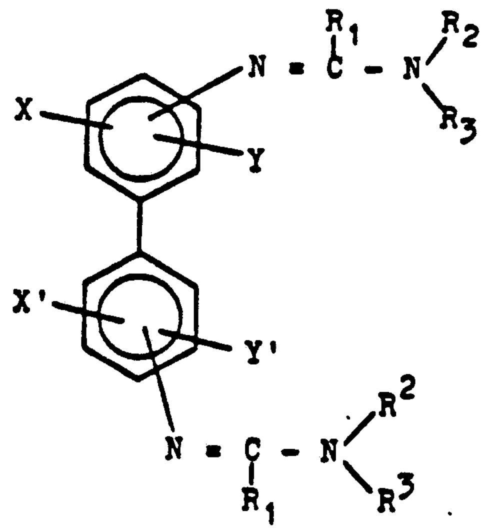

(I)

dans laquelle

| | |
|---|---|
| X et X' | représentent chacun un radical alkyle $C_1$-$C_5$, alcoxy $C_1$-$C_5$, un halogène ou un groupe $SO_2CH_3$, |
| Y et Y' | représentent chacun l'hydrogène ou un halogène, |
| $R_1$ | représente l'hydrogène, un radical alkyle $C_1$-$C_5$ ou alkyle substitué de formule $(CH_2)_nXR_4$, n représentant un nombre entier de 1 à 3, X représentant O ou S et $R_4$ représentant l'hydrogène, un radical alkyle $C_1$-$C_3$ ou alcanoyle $C_1$-$C_5$, |
| $R_2$ et $R_3$ | sont identiques ou différents et représentent chacun l'hydrogène, un radical alkyle $C_1$-$C_5$, alcanoyle $C_1$-$C_5$ ou alkyle substituè de formule $(CH2)_nXR_4$, n représentant un nombre entier de 1 à 3, X représentant O ou S et $R_4$ représentant l'hydrogène, un radical alkyle $C_1$-$C_3$ ou alcanoyle $C_1$-$C_5$, |
| $R_2$ et $R_3$ | représentent, avec l'atome de N auquel ils sont liés, le radical pyrrolidino, pipéridino, imidazolino ou pyrimidino, ou |
| $R_1$ et $R_2$ | représentent, avec l'atome de carbone et l'atome d'azote auxquels ils sont liés, le radical pyrrolidino, pipéridino, imidazolino ou pyrimidino, qui peut être substitué une fois ou plusieurs fois par un radical alcanoyle $C_1$-$C_5$, alkyle $C_1$-$C_5$ ou phényle non substitué ou substitué par un halogène, $NO_2$, CN, $CF_3$ ou $SO_3H$, |
| $R_3$ | représente l'hydrogène, un radical alkyle $C_1$-$C_5$, alkyle substitué de formule $(CH_2)_nXR_4$, n représentant un nombre entier de 1 à 3, X représentant O ou S et $R_4$ représentant l'hydrogène, un radical alkyle $C_1$-$C_3$ ou alcanoyle $C_1$-$C_5$, un radical alcanoyle $C_1$-$C_5$, carbalcoxy $C_2$-$C_5$ ou phényle non substitué ou substitué par un halogène, $NO_2$, CN, $CF_3$ ou $SO_3H$, |

leurs stéréoisomères et leurs sels pharmaceutiquement acceptables.

2. Composés selon la revendication 1 de formule générale I'

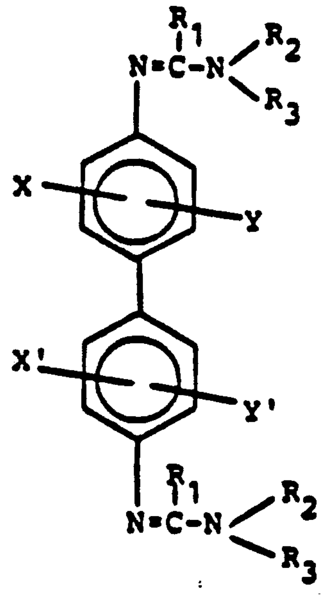

(I')

dans laquelle X,X', Y,Y', $R_1$, $R_2$ et $R_3$ ont la signification indiquée dans la revendication 1.

3. 3,3'-dichloro-4,4'-di(pyrrolidine-2-ylidène-amino)-diphényle.

4. 3,3'-difluoro-4,4'-di(pyrrolidine-2-ylidène-amino)-diphényle.

5. 3,3'-difluoro-4,4'-di(5-méthylpyrrolidine-2-ylidèneamino)-diphényle.

6. Procédé de préparation de composés de formule générale I selon la revendication 1 caractérisé en ce que l'on fait réagir un composé de formule générale II

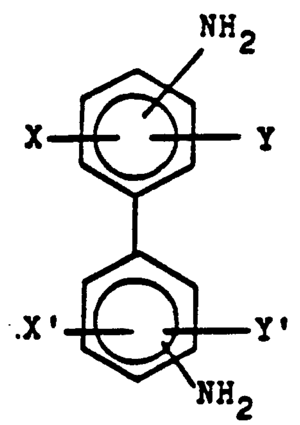

(II)

dans laquelle X, X', Y et Y' ont la signification indiquée dans la revendication 1, avec de l'oxychlorure de phosphore et un amide de formule generale III

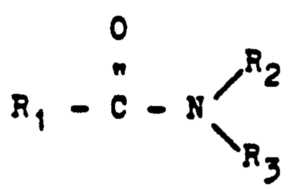

(III)

dans laquelle $R_1$, $R_2$ et $R_3$ ont la signification indiquée dans la revendication 1, ce qui donne un composé de formule générale I et en ce que l'on convertit éventuellement celui-ci en son sel d'addition d'acide pharmaceutiquement acceptable.

7. Procédé selon la revendication 6, caractérisé en ce que le mélange de réaction est chauffé à 60 - 80°C.

8. Procédé selon la revendication 7, caractérisé en ce que la réaction est effectuée dans un hydrocarbure halogéné ou dans un éther.

9. Médicament caractérisé en ce qu'il contient un composé de formule générale I ou un sel d'addition d'acide pharmaceutiquement acceptable de celui-ci associé à un véhicule et/ou un stabilisant pharmaceutique courant.

10. Emploi d'un composé selon la revendication 1 pour la préparation d'un médicament actif contre les amibes et Trichomonas.

**Revendications** pour l'Etat contractant: AT

1. Procédé de préparation de composés de formule générale I

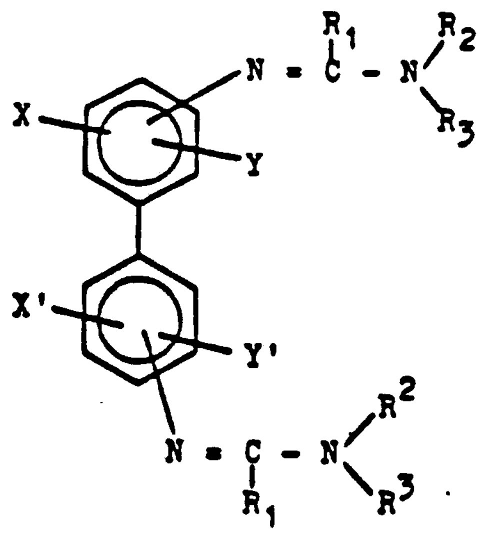

(I)

dans laquelle

| | |
|---|---|
| X et X' | représentent chacun un radical alkyle $C_1$-$C_5$, alcoxy $C_1$-$C_5$, un halogène ou un groupe $SO_2CH_3$, |
| Y et Y' | représentent chacun l'hydrogène ou un halogène, |
| $R_1$ | représente l'hydrogène, un radical alkyle $C_1$-$C_5$ ou alkyle substitué de formule $(CH2)_nXR_4$, n représentant un nombre entier de 1 à 3, X représentant O ou S et $R_4$ représentant l'hydrogène, un radical alkyle $C_1$-$C_3$ ou alcanoyle $C_1$-$C_5$, |
| $R_2$ et $R_3$ | sont identiques ou différents et représentent chacun l'hydrogène, un radical alkyle $C_1$-$C_5$, alcanoyle $C_1$-$C_5$ ou alkyle substitué de formule $(CH_2)_nXR_4$, n représentant un nombre entier de 1 à 3, X représentant O ou S et $R_4$ représentant l'hydrogène, un radical alkyle $C_1$-$C_3$ ou alcanoyle $C_1$-$C_5$, |
| $R_2$ et $R_3$ | représentent, avec l'atome de N auquel ils sont liés, le radical pyrrolidino, pipéridino, imidazolino ou pyrimidino, ou |
| $R_1$ et $R_2$ | représentent, avec l'atome de carbone et l'atome d'azote auxquels ils sont liés, le radical pyrrolidino, pipéridino, imidazolino ou pyrimidino, qui peut être substitué une fois ou plusieurs fois par un radical alcanoyle $C_1$-$C_5$, alkyle $C_1$-$C_5$ ou phényle non substitué ou substitué par un halogène, $NO_2$, CN, $CF_3$ ou $SO_3H$, |
| $R_3$ | représente l'hydrogène, un radical alkyle $C_1$-$C_5$, alkyle substitué de formule $(CH_2)_nXR_4$, n représentant un nombre entier de 1 à 3, X représentant O ou S et $R_4$ représentant l'hydrogène, un radical alkyle $C_1$-$C_3$ ou alcanoyle $C_1$-$C_5$, un radical alcanoyle $C_1$-$C_5$, carbalcoxy $C_2$-$C_5$ ou phényle non substitué ou substitué par un halogène, $NO_2$, CN, $CF_3$ ou $SO_3H$, |

de leurs stéréoisomères et de leurs sels pharmaceutiquement acceptables caractérisé en ce que l'on fait réagir un composé de formule générale II

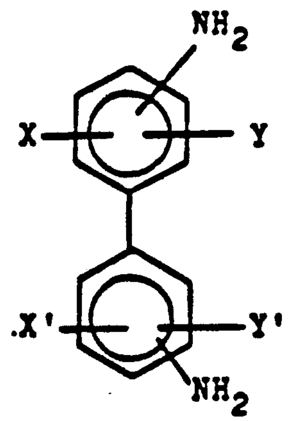

(II)

dans laquelle
X,X', Y et Y' ont la signification indiquée ci- dessus, avec de l'oxychlorure de phosphore et un amide de formule générale III

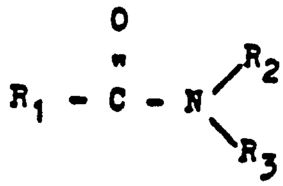

(III)

dans laquelle $R_1$, $R_2$ et $R_3$ ont la signification indiquée ci-dessus, ce qui donne un composé de formule générale I et en ce que l'on convertit éventuellement celui-ci en son sel d'addition d'acide pharmaceutiquement acceptable.

2. Procédé selon la revendication 1, caractérisé en ce que le mélange de réaction est chauffé à 60 - 80° C.

3. Procédé selon la revendication 2, caractérisé en ce que la réaction est effectuée dans un hydrocarbure halogéné ou dans un éther.

4. Procédé selon les revendications 1 à 3, caractérisé en ce que l'on prépare un composé de formule générale I'

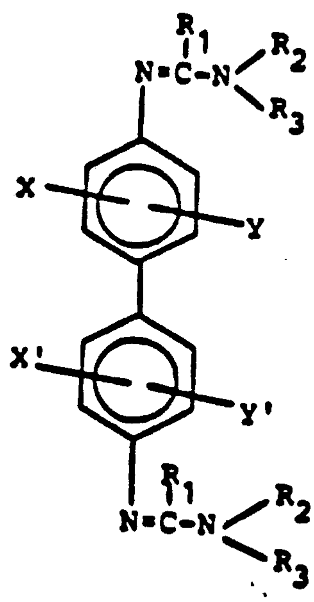

(I')

dans laquelle
X,X', Y,Y', $R_1$, $R_2$ et $R_3$ ont la signification indiquée dans la revendication 1.

5. Procédé selon la revendication 4, caractérisé en ce que l'on prépare le 3,3'-dichloro-4,4'-di-(pyrrolidine-2-ylidèneamino)-diphényle.

6. Procédé selon la revendication 4, caractérisé en ce que l'on prépare le 3,3'-difluoro-4,4'-di-(pyrrolidine-2-ylidèneamino)-diphényle.

7. Procédé selon la revendication 4, caractérisé en ce que l'on prépare le 3,3'-difluoro-4,4'-di(5-méthyl-pyrrolidine-2-ylidèneamino)-diphényle.

8. Procédé de préparation de médicaments, caractérisé en ce que l'on amène un composé de formule générale I ou un sel d'addition d'acide pharmaceutiquement acceptable de celui-ci, avec des véhicules et/ou des stabilisants pharmaceutiques courants à une forme d'administration thérapeutiquement appropriée.

9. Emploi d'un composé selon la revendication 1 pour la préparation d'un médicament actif contre les amibes et Trichomonas.